# EUROPEAN PATENT SPECIFICATION

(11) **EP 2 770 975 B1**
(45) Date of publication and mention of the grant of the patent: **29.08.2018**
(21) Application number: 12780469.8
(22) Date of filing: 22.10.2012
(51) Int. Cl.: A61K 8/41, A61K 8/42, A61K 8/44, A61K 8/46, A61Q 5/00, A61Q 5/12, A61Q 5/02

(54) **CLEANSING COMPOSITION**
REINIGUNGSMITTELZUSAMMENSETZUNG
COMPOSITION DE NETTOYAGE

(30) Priority: 25.10.2011 EP 11186581
(43) Date of publication of application: 03.09.2014
(73) Proprietor: Kao Germany GmbH, 64297 Darmstadt (DE)
(72) Inventor: HOFFMANN, Martin, 64673 Zwingenberg (DE); OLSSON, Kristin, 68526 Ladenburg (DE)
(74) Representative: Grit, Mustafa
(86) International application number: PCT/EP2012/070888
(87) International publication number: WO 2013/060651

(56) References cited:
- EP-A1- 1 547 574
- EP-A1- 1 925 290
- EP-A1- 2 161 016
- EP-A1- 2 184 051
- EP-A1- 2 335 679
- EP-A1- 2 380 555
- US-A1- 2008 311 068
- "Shampoo", GNPD, 1 November 2009 (2009-11-01), XP055023733, Retrieved from the Internet: URL:http://www.gnpd.com/sinatra/gnpd/searc h_results&search_id=1219658 [retrieved on 2012-04-03]
- "Daily Shampoo", GNPD, 1 March 2003 (2003-03-01), XP055023742, Retrieved from the Internet: URL:http://www.gnpd.com/sinatra/gnpd/searc h_results&search_id=z5WJkWZNyf/&s_item=sea rch_results&item_id=244802 [retrieved on 2012-04-03]
- "Shampoo DM Réell'e Brown", GNPD, 1 April 2008 (2008-04-01), XP055023746, Retrieved from the Internet: URL:http://www.gnpd.com/sinatra/gnpd/searc h_results&search_id=z5WJkWZNyf/&s_item=sea rch_results&item_id=904880 [retrieved on 2012-04-03]

## Description

Present invention relates to an aqueous cleansing composition for keratin fibres, especially human hair, comprising at least one amino acid surfactant and at least one cationic surfactant and process for treating artificially coloured hair.

Cleansing compositions have been subject matter of many patent applications. The problem addressed is unsatisfactory conditioning and cleansing with existing technologies. Although plurality of the solutions suggested for improved cleansing and conditioning, there is still need for further improvement, especially in terms of foaming power, in particular foam creaminess, and conditioning effect on hair.

Furthermore, cleansing compositions used for cleansing artificially coloured hair wash out colour pigments from hair so that hair losses its colour intensity in a relatively short period of time. There have been many attempts to optimize the colour wash out effect of cleansing composition which have provided limited successful solutions. Products available on the market designed for coloured hair and claiming that using them on artificially coloured hair extends the lifetime of artificial hair colour fail to prove the claimed benefit. Therefore, there is still great need for cleansing composition which washes significantly less colour pigments from artificially coloured hair and thus, hair colour has significantly longer life time as it keeps its colour intensity, colour tone and shine significantly for longer period of time.

Present invention starts from the above mentioned problems and aims at providing an aqueous cleansing composition especially for artificially coloured hair and a process for treating artifically coloured hair which has excellent foam and hair conditioning properties and especially importantly it washes significantly less hair colour so that colour intensity, tone and shine are kept for a longer period of time.

EP 2184051 A1 discloses cleansing compositions comprising amino acid surfactants which wash out less colour from artifically coloured hair. With the term hair conditioning it is meant that after washing hair with aqueous cleansing composition of the present invention hair is easily combable, hair feels soft and smooth and has improved elasticity, volume and body.

With the term foam properties, it is meant that the cleansing composition produces satisfactory volume of foam and the foam has a creamy and pleasant feeling.

Present inventors have surprisingly found that an aqueous cleansing composition comprising at least one amino acid surfactant and at least one cationic surfactant has excellent foam properties, provides excellent conditioning effect to human hair and has extremely reduced colour wash out effect. The colour wash out effect is so reduced that in some of cases the results obtained have been found to be comparable for coloured hair tresses washed only with water.Accordingly, the first object of the present invention is an aqueous cleansing composition comprising at least one amino acid surfactant selected from glutamate surfactants according to the general formula 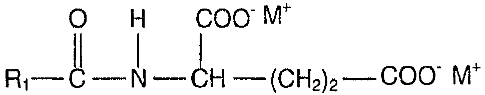 and
sarcosinate surfactants according to the general formula 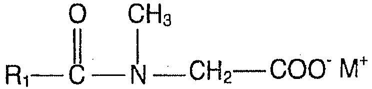 wherein R₁ is saturated or unsaturated, straight or branched alkyl chain with 7 to 17 C atoms, and M is independent from each other H, sodium or potassium at a concentration of 0.25 to 15% by weight, calculated to total composition, and at least one cationic surfactant of the general structure 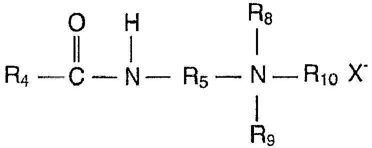 wherein R₄ is a saturated or unsaturated, straight or branched alkyl chain with 8 to 24 C atoms, R₅ is a straight or branched alkyl chain with 1 to 4 C atoms,
R₈ and R₉ are the same or different, an alkyl chain with 1 to 4 C atoms, hydroxyl alkyl chain with 1 to 4 C atoms and di hydroxyl alkyl chain with 2 to 4 C atoms, R₁₀ is an alkyl chain with 1 to 4 C atoms, hydroxyl alkyl chain with 1 to 4 C atoms or di hydroxyl alkyl chain with 2 to 4 C atoms and

-R₅-A-R₄

wherein R₄ and R₅ have the above meaning, A is 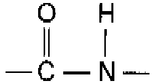 and X is an anion selected from chloride, bromide and methosulfate at a concentration of 0.01 to 5% by weight, calculated to total composition.

Further object of the present invention is process for treating hair wherein hair is treated with a composition comprising at least one hair dyestuff and subsequently optionally washed with a cleansing composition and afterwards treated with an aqueous cleansing composition of the present invention as outlined above.

Aqueous cleansing compositions of the present invention comprise surfactants at a total concentration of 5 to 50%, preferably 7.5 to 40% and more preferably 10 to 30%, and most preferably 10 to 20% by weight, calculated to the total composition.

Aqueous cleansing composition of the present invention comprises at least one amino acid surfactant according to the general structure given above preferably at a concentration of 0.25 to 15%, by weight, calculated to total composition. More preferably, the concentration of amino acid surfactant is from 0.5 to 10% by weight, further preferably 1 to 7.5% by weight and most preferably 1.75 to 5% by weight, calculated to total composition. The concentrations mentioned here are total concentration ranges in case more than one amino acid surfactant is present in the composition. In the preferred embodiment of the present invention R₁ in the general formulas of amino acid surfactants disclosed above is a saturated or unsaturated, straight or branched alkyl chain with 9 to 17 C atoms, and more preferably 9 to 13 C atoms, R₂ is H or a methyl, R₃ is H, COO⁻ M⁺, CH₂COO⁻ M or COOH, n is 0 to 2, X is COO⁻ or SO₃⁻ and M is independent from each other H, ammonium, sodium or potassium. It should be noted that alkyl chain includes also mixture of various alkyl chains as present especially in plant triglyceride derived alkyl chains such as cocoyl chain.

Suitable glutamate surfactants are dipotassium capryloyl glutamate, dipotassium undecylenoyl glutamate, disodium capryloyl glutamate, disodium cocoyl glutamate, disodium lauroyl glutamate, disodium stearoyl glutamate, disodium undecylenoyl glutamate, potassium capryloyl glutamate, potassium cocoyl glutamate, potassium lauroyl glutamate, potassium myristoyl glutamate, potassium stearoyl glutamate, potassium undecylenoyl glutamate, sodium capryloyl glutamate, sodium cocoyl glutamate, sodium lauroyl glutamate, sodium myristoyl glutamate, sodium olivoyl glutamate, sodium palmitoyl glutamate, sodium stearoyl glutamate, and sodium undecylenoyl glutamate and mixtures thereof. Preferred are disodium capryloyl glutamate, disodium cocoyl glutamate, disodium lauroyl glutamate, potassium capryloyl glutamate, potassium cocoyl glutamate, potassium lauroyl glutamate, potassium myristoyl glutamate, sodium capryloyl glutamate, sodium cocoyl glutamate, sodium lauroyl glutamate, and sodium myristoyl glutamate and mixtures thereof. More preferred are disodium cocoyl glutamate, disodium lauroyl glutamate, potassium cocoyl glutamate, potassium lauroyl glutamate, sodium cocoyl glutamate, and sodium lauroyl glutamate and mixtures thereof.

Suitable sarcosinate surfactants are potassium lauroyl sarcosinate, potassium cocoyl sarcosinate, sodium cocoyl sarcosinate, sodium lauroyl sarcosinate, sodium myristoyl sarcosinate, and sodium palmitoyl sarcosinate and mixtures thereof. Preferred are ammonium lauroyl sarcosinate, ammonium cocoyl sarcosinate, potassium lauroyl sarcosinate, potassium cocoyl sarcosinate, sodium cocoyl sarcosinate, and sodium lauroyl sarcosinate and mixtures thereof. More preferred are sodium cocoyl sarcosinate, and sodium lauroyl sarcosinate and mixtures thereof.

In a preferred form of the present invention, aqueous cleansing compositions comprise the sarcosinate and glutamate surfactants at a weight ratio of sarcosinate to glutamate surfactants in a range of 1:1 to 10:1, preferably 1:1 to 5 to 1 and more preferably 2:1 to 4:1.
In a further preferred embodiment of the invention, total amino acid surfactant content of the aqueous cleansing composition is always higher than total cationic surfactant content. More specifically, the weight ratio of the total amino acids surfactant concentration and total cationic surfactant concentration is higher than 1, preferably at least 1.5, more preferably at least 2 and most preferably at least 3.
Compositions of the present invention comprise at least one cationic surfactant according to the above general structures. In the preferred embodiment of the present invention, R₄ is saturated or unsaturated, straight or branched alkyl chain with 10 to 24C atoms, more preferably 12 to 22 C atoms and R₅ is straight or branched alkyl group with 1 to 4 C atoms, A, B, R₆ to R₁₀ are same as above. Non-limiting suitable examples are, palmitoamidopropyl trimethyl ammonium chloride, stearamidopropyl trimethylammonium chloride, behenamidopropyl tri hydroxyethylammonium chloride and distearylamidopropyl dimethyl ammonium chloride. The most preferred compounds are palmitamidopropyl trimonium chloride and stearamidopropyltrimonium chloride.
Concentration of at least one cationic surfactant according to the above general structure is in the range of 0.01 to 5%, preferably 0.02 to 4%, more preferably 0.05 to 3% and most preferably 0.1 to 2% and in particular 0.25 to 1% by weight calculated to total composition.
Preferably aqueous cleansing compositions of the present invention comprise at least one anionic surfactant in addition to the amino acid surfactants at a concentration range of 2 to 40%, preferably 3 to 30% and more preferably 5 to 20%, and most preferably 5 to 15% by weight, calculated to the total composition.
Within the scope of the present invention, with the term anionic surfactant it is meant any anionic surfactant other than amino acid surfactants.
In principal any anionic surfactant is suitable within the meaning of the present invention. As mentioned above with the term anionic surfactant any anionic surfactants are meant other than amino acid surfactants. Nonlimiting examples are anionic surfactants of the sulfate, sulfonate, carboxylate and alkyl phosphate type, especially, of course, those customarily used in shampoo compositions, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates constituting mild, skin-compatible detergents.
The alkyl ether surfactants preferred as an additional anionic surfactants within the meaning of the present invention are according to the general structure

R₁₁(OCH₂CH₂)ₙOSO₃⁻M⁺

wherein R₁₁ is a straight or branched, saturated or unsaturated alkyl chain with 10 to 24 C atoms, preferably 10 to 18 C atoms, more preferably 10 to 16 C atoms and most preferably 12 to 14 C atoms, n is a value between 1 and 5 and M is a cation such as ammonium, sodium, potassium, magnesium. Suitable and most preferred is sodium laureth sulphate available under the trade name Texapon from Cognis or Emal from Kao Corporation.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₁₂-(C₂H₄O)ₙ-O-CH₂COOX,

wherein R₁₂ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 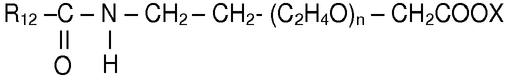 wherein R₁₂ and X have the above meanings, and n is in particular a number from 1 to 10, preferably 2.5 to 5.

Such products have been known for some time and are on the market, for example, under the trade name "AKYPO®" and "AKYPO-SOFT®".

Also useful are C₈-C₂₀-acyl isethionates, alone or in admixture with other anionic surfactants, as well as sulfofatty acids and the esters thereof.

It is also possible to use mixtures of several anionic surfactants, for example an ether sulfate and a polyether carboxylic acid or alkyl amidoether carboxylic acid.

The most preferred anionic surfactants within the meaning of the present invention are those of alkyl ether sulphates according to above general structure such as lauryl ether sulphate sodium salt.

In a preferred embodiment of the present invention, cleansing composition of the present invention comprises at least one anionic surfactant as mentioned above and at least one nonionic surfactant. Nonionic surfactants are suitable at a concentration of 1 to 15%, in particular from 1 to 10% by weight, calculated to the total composition.

Nonionic surfactants especially suited in the cleansing compositions according to the invention are alkyl polyglucosides of the general formula

R₁₃-O-(R₁₄O)ₙ-Zₓ,

wherein R₁₃ is an alkyl group with 8 to 18 carbon atoms, R₁₄ is an ethylene or propylene group, Z is a saccharide group with 5 to 6 carbon atoms, n is a number from 0 to 10 and x is a number between 1 and 5.

These alkyl polyglucosides are known in particular as excellent skin-compatible, foam improving agents in liquid detergents and body cleansing compositions. Mixtures of anionic surfactants and alkyl polyglucosides as well as the use thereof in liquid body cleansing compositions are already known, for example, from EP-A 70 074. The alkyl polyglucosides disclosed therein are basically also suited within the scope of the present invention; as well as the mixtures of sulfosuccinates and alkyl polyglucosides disclosed in EP-A 358 216.

Further nonionic surfactants are, suitable for the cleansing compositions of the present invention, long-chain fatty acid dialkanolamides, such as coco fatty acid diethanolamide and myristic fatty acid diethanolamide.

Further suitable nonionic surfactants are amineoxides. Such amineoxides are state of the art, for example C₁₂-C₁₈-alkyl dimethyl amineoxides such as lauryl dimethyl amineoxide, C₁₂-C₁₈-alkyl amidopropyl or -ethyl amineoxides, C₁₂-C₁₈-alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx®", "Aromox®" or "Genaminox®".

Further nonionic surfactants useful in the compositions according to invention are C₁₀-C₂₂-fatty alcohol ethoxylate. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":
The average degree of ethoxylation thereby ranges between about 2.5 and about 25, preferably about 10 and about 20.

Further nonionic surfactants are sorbitan surfactants suitable within the meaning of the present invention. Suitable non-limiting examples are esters of sorbitan such as sorbitan caprylate, sorbitan cocoate, sorbitan diisostearate, sorbitan dioleate, sorbitan distearate, sorbitan isostearate, sorbitan laurate, sorbitan oleate, sorbitan olivate, sorbitan palmitate, sorbitan palmate, sorbitan sesquicaprylate, sorbitan sesquiisostearate, sorbitan sesquioleate, sorbitan sesquistearate and sorbitan stearate, as well as ethoxylated sorbitan surfactants such as PEG-20 sorbitan cocoate, PEG-40 sorbitan diisostearate, PEG-2 sorbitan isostearate, PEG-5 sorbitan isostearate, PEG-20 sorbitan isostearate, PEG-40 sorbitan laurate, PEG-10 sorbitan laurate, PEG-44 sorbitan laurate, PEG-75 sorbitan laurate, PEG-80 sorbitan laurate, PEG-3 sorbitan oleate, PEG-6 sorbitan oleate, PEG-20 sorbitan oleate, PEG-40 sorbitan oleate, PEG-80 sorbitan palmitate, PEG-3 sorbitan stearate, PEG-4 sorbitan stearate, PEG-6 sorbitan stearate, PEG-40 sorbitan stearate, and PEG-60 sorbitan stearate, and as well as Polysorbate 20, Polysorbate 21, Polysorbate 40, Polysorbate 60, Polysorbate 61, Polysorbate 65, Polysorbate 80, Polysorbate 81, and Polysorbate 85. Preferred are sorbitan caprylate, sorbitan cocoate, sorbitan diisostearate, sorbitan dioleate, sorbitan distearate, sorbitan isostearate, sorbitan laurate, sorbitan oleate, sorbitan olivate, sorbitan palmitate, sorbitan palmate, sorbitan sesquicaprylate, sorbitan sesquiisostearate, sorbitan sesquioleate, sorbitan sesquistearate, sorbitan stearate, PEG-20 sorbitan cocoate, PEG-40 sorbitan diisostearate, PEG-2 sorbitan isostearate, PEG-5 sorbitan isostearate, PEG-20 sorbitan isostearate, PEG-40 sorbitan laurate, PEG-10 sorbitan laurate, PEG-44 sorbitan laurate, PEG-75 sorbitan laurate, PEG-80 sorbitan laurate, PEG-3 sorbitan oleate, PEG-6 sorbitan oleate, PEG-20 sorbitan oleate, PEG-40 sorbitan oleate, PEG-80 sorbitan palmitate, PEG-3 sorbitan stearate, PEG-4 sorbitan stearate, PEG-6 sorbitan stearate, PEG-40 sorbitan stearate, and PEG-60 sorbitan stearate.

The most preferred non-ionic surfactants are alkyl polyglucosides such as decyl, cocoyl polyglucoside and ethoxylated fatty alcohols such as laureth-16.

In a further preferred embodiment of the present invention, cleansing composition of the present invention comprises at least one anionic, at least one nonionic surfactant and at least one amphoteric or zwitterionic surfactant.

Amphoteric or zwitterionic surfactants, are present at a concentration of 0.5 % to about 15 %, preferably 1 % to about 10 %, by weight, calculated to the total composition. It has especially been found out that addition of zwitterionic or amphoteric surfactants enhances foam feeling in terms of creaminess, foam volume and as well as skin compatibility are also improved. For achieving milder formulations anionic surfactant, especially of sulphate types, to amphoteric surfactant ratio should be in the range of 10:1 to 1:1, preferably 5:1 to 1:1.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, suitable betaine surfactants are of general structure 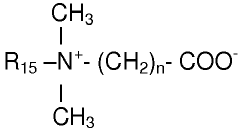 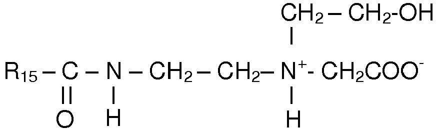 wherein R₁₅ is a C₈-C₁₈-alkyl group and n is 1 to 3;
sulfobetaines of the structure 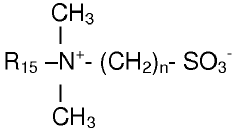 wherein R₁₅ and n are same as above;
hydroxysulfobetaines of the structure 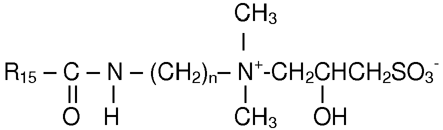 wherein R₁₅ and n are same as above;
and amidoalkyl betaines of the structure 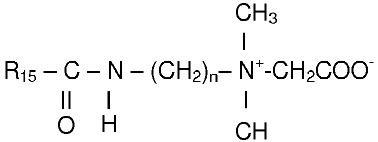 wherein R₁₅ and n are same as above.

The most preferred amphoteric surfactants are alkyl betaines such as lauryl betaine and alkyl amido betaines such as cocamidopropyl betaine and hydroxysulphobetaines such as cocamidopropyl hydroxysultaine.

In the most preferred embodiment of the present invention, aqueous cleansing composition comprises at least one anionic surfactant especially of alkyl ether sulphate type, at least one amphoteric surfactant especially alkyl amido alkyl betaine type and/or alkylamidoalkyl hydroxysultaine type and at least one non-ionic surfactant especially an alkyl polyglucoside type in the above mentioned concentration ranges, certainly in addition to the amino acid surfactant and cationic and/or cationizable surfactant.

Aqueous cleansing composition of the present invention preferably comprises hair-conditioning agents. Conditioning agents can be selected from oily substances, non-ionic substances and cationic polymers or their mixtures.

Oily substances are selected from such as silicone oils, either volatile or non-volatile, natural and synthetic oils. Among silicone oils those can be added to the compositions include dimethicone, dimethiconol, polydimethylsiloxane, DC fluid ranges from Dow Corning, arylated silicones such as phenyl trimethicone or any other silicone with up to 5 aryl, preferably phenyl, group in its molecule such as trimethyl pentaphenyl trisiloxane, natural oils such as olive oil, almond oil, avocado oil, wheatgerm oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Non-ionic conditioning agents can be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₁₉CO(OCH₂CH₂)ₙOH

or

R₁₉CO(OCH₂CH₂)ₙOOCR₂₀

where R₁₉ and R₂₀ are independent from each other saturated, unsaturated or branched or non-branched alkyl chain with 7 to 21 C atoms and n is typically 2 - 100.

In one of the preferred form of the present invention, aqueous cleansing compositions comprise at least one additional cationic polymer as a conditioning agent. Suitable cationic polymers are those of best known with their CTFA category name Polyquaternium. Typical examples of those are Polyquaternium 1, Polyquaternium 2, Polyquaternium 4, Polyquaternium 5, Polyquaternium 6, Polyquaternium 7, Polyquaternium 8, Polyquaternium 9, Polyquaternium 10, Polyquaternium 11, Polyquaternium 12, Polyquaternium 13, Polyquaternium 14, Polyquaternium 15, Polyquaternium 16, Polyquaternium 17, Polyquaternium 18, Polyquaternium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquaternium-49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquaternium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, Polyquaternium 86 and Polyquaternium 87.

Additionally cationic starch derived polymers especially oxidized cationic starch polymer known with it CTFA/INCI name hydroxypropyl Oxidized Starch PG-Trimonium chloride available from Graefe Chemie GmbH under the trade name Amylomer are comprised in the aquoues cleansing compositions of the present invention.

Furthermore, cationic polymers known with the CTFA/INCI adopted name Silicone Quaternium are also preferred cationic polymers. Expecially preferred is Silicone quaternium 22.

It has further been found out that especially those of cationic cellulose type polymers known as Polymer JR type from Amerchol such as Polyquaternium 10 or cationic galactomannans such as cationic guar gum known with trade name Jaguar from Rhône-Poulenc which are chemically for example Guar hydroxypropyl trimonium chloride and cationic tara gum an its derivatives known with INCI name Caesalpinia spinosa hydroxypropyltrimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, cationic Caesalpinia spinosa gum derivatives, polyquaternium 6, polyquaternium 7, polyquaternium 67 and polyquaternium 70.

The cationic polymers also include the quaternized products of graft polymers from organopolysiloxanes and polyethyl oxazolines described in EP-A 524 612 and EP-A 640 643.

Total concentration of conditioning agents mentioned above namely oily substances, non-ionic substances and cationic polymers in the compositions is in the range of 0.01 to 5%, preferably 0.02 to 4%, more preferably 0.05 to 3% and most preferably 0.1 to 2.5% by weight calculated to total composition. Especially preferred are the cationic polymers as the conditioning agents in the given concentration ranges.

The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Aqueous cleansing composition may comprise at least one organic solvent such as ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, propyleneglycol, polypropyleneglycols, ethyleneglycol monoethylether, ethylene glycol monobutyl ether, ethylene glycol monophenyl ether, 1-phenylethylalcohol, 2-phenylethylalcohol, o-methoxyphenol. The most preferred ones are benzyl alcohol and polypropylene glycols. Total concentration of organic solvents in the shampoo composition should not exceed 5% by weight, preferably in the range of 0.1 to 3%, more preferably 0.5 to 2.5% by weight calculated to total composition.

Aqueous cleansing composition of the present invention may further comprise at least one fatty alcohol of the following formula

R₂₁-OH

wherein R₂₁ is straight or branched, saturated or unsaturated alkyl chain with 8 to 24, preferably 10 to 22, more preferably 12 to 18 and most preferably 12 to 16C atoms at a concentration of 0.1 to 5%, preferably 0.1 to 4% and more preferably 0.25 to 3% and most preferably 0.5 to 2.5% by weight calculated to total composition.

Suitable non-limiting preferred examples are decyl alcohol, myristyl alcohol, lauryl alcohol, cetyl alcohol, stearyl alcohol, behenyl alcohol, and arachidyl alcohol and their mixtures. More preferred are decyl alcohol, myristyl alcohol, lauryl alcohol, cetyl alcohol, and stearyl alcohol. Most preferred are decyl alcohol, myristyl alcohol and lauryl alcohol, and their mixtures.

Cleansing composition of the present invention may be pearly. Pearl-shiny appearance is achieved with those dispersed in cleansing conditioning compositions in crystalline form, i.e. so called pearl-shine or pearlizing agents. The preferred once are PEG-3 distearate and ethylene glycol distearate. The concentration of those can typically be from 0.1 to 3%, preferably 0.5 to 2% by weight, calculated to the total composition. These compounds are preferably added to the compositions in admixture with anionic, nonionic and/or amphoteric surfactants. Such kinds of mixtures are available commercially.

Solubilizers may be added to the compositions especially when oily substances are chosen as conditioning agents and fragrance oils with highly lipophilic properties. Typical solubilizers may be hydrogenated castor oil known with the trade mark Cremophor CO series from BASF. It should be noted that as well the surfactant mixture can be a good solubilizer for fragrance oils. Typical concentration of the solubilizers can be in the range of 0.01 - 2% by weight, preferably 0.1 - 1% by weight, calculated to total composition.

The cleansing composition may contain active ingredients selected from UV filters, moisturisers, sequestering agents, and natural ingredients.

The moisturizing agents are selected from panthenol, polyols, such as glycerol, polyethylene glycols with molecular weight 200 to 20,000. The moisturizing ingredients can be included in the conditioner compositions at a concentration range of 0.01 - 2.5% by weight calculated to the total composition.

The sequestering agents are preferably selected from polycarboxy acids. The preferred one is ethylene diamine tetraacetic acid, EDTA. Typical useful concentration range for sequestering agents is of 0.01 - 2.5% by weight calculated to the total composition.

The UV filters are that oil and water soluble ones for the purpose of protecting hair and hair colour. In other words, anionic and non-ionic, oily, UV filters are suitably used in the compositions of the present invention. Suitable UV-absorbing substances is are: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxybenzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof, 3-(4'-methyl benzylidene)-DL-campher, and/or polysilicone-15. The amount of the UV-absorber ranges typically from about 0.01 % to 2.5%, more preferably from 0.05 % to 1 % by weight, calculated to the total composition.

Natural plant extracts are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known **per se** are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Suitable trade products are, for example, various "Extrapone®" products, and "Herbasol^{R}". Extracts and the preparation thereof are also described in "Hagers Handbuch der pharmazeutischen Praxis", 4th Ed..

Compositions of the present invention may comprise further at least one compound according to the formula 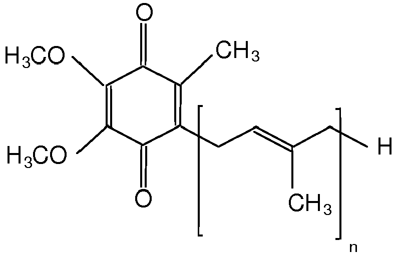 where n is a number from 1 to 10.

The compounds of the above formula are known as Ubiquinone, and also are known as Coenzyme. It should be noted that the compositions of the present invention can certainly comprise more than one ubichinone. Preferred ubichinones are the ones where n is a number between 6 and 10 and especially preferred is Ubichinone 50 where n is 10, also known as Coenzyme Q10. Concentration ubichinone of the above formula in the compositions is from 0.0001 to 1%, preferably from 0.0002 to 0.75%, more preferably from 0.0002 to 0.5% and most preferably from 0.0005 to 0.5% by weight, calculated to total composition.

Aqueous cleansing compositions may further comprise at least one direct dye. Suitable direct dyes are of cationic, anionic and neutral nitro dyes. It should be noted that they can also be used in combination with each other. In other words a composition according to present invention can comprise an anionic and a cationic dye as well as an anionic and a nitro dye or a cationic and a nitro dye. Certainly the combination of all three dyestuff categories is also possible.

Any cationic direct dye is in principal suitable for the compositions. Examples are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87, and mixtures thereof

Any anionic dye is in principal suitable for the compositions. Suitable examples are such as Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium, and mixtures thereof.

Among those, the preferred anionic dyestuffs are Acid Red 52, Acid Violet 2, Acid Red 33, Acid Orange 4, Acid Red 27 and Acid Yellow 10 and their salts, and mixtures thereof. The most preferred anionic dyes are Acid Red 52, Acid Violet 2, Acid Red 33, Acid Orange 4 and Acid Yellow 10, and their salts, and mixtures thereof.

Neutral dyes, so called nitro dyes for shading purposes are also optionally contained in the compositions. Suitable ones are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid, and mixtures thereof.

Concentration of one or more direct dyes in total is in the range of 0.001 to 5% by weight, preferably 0.01 to 4% more preferably 0.05 to 3% and most preferably 0.1 to 2.5% by weight calculated to total composition. The most preferred among the direct dyes is cationic direct dyes.

It is self-understood that the shampoos according to the invention may comprise other substances customarily used in such compositions such as preservatives, fragrances.

The pH of the compositions according to the present invention is suitably between 2 and 8.0, preferably in the range of 2.5 to 7.0, more preferably 3 to 6.5 and most preferably 4 to 5.5 measured at ambient temperature with a suitable pH meter.

pH of the compositions is adjusted with acidic and alkaline compounds. Acidic compounds can be inorganic and organic acid or their mixtures. Nonlimiting suitable examples are citric acid, lactic acid, glycolic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid. Alkaline compounds such as sodium hydroxide can be used to adjust the pH of the compositions.

Aqueous cleansing composition of the present invention preferably comprises one or more thickeners. Suitable ones are ethoxylated polyglyceryl esters with total ethoxy units in the range of 50 to 200 and fatty acyl chain length of 8 to 22 C atoms such as PEG-80 glyceryl cocoate, PEG-90 glyceryl isostearate, PEG-120 glyceryl stearate, PEG-200 glyceryl stearate, PEG-80 glyceryl tallowate, PEG-82 glyceryl tallowate, PEG-130 glyceryl tallowate, and PEG-200 glyceryl tallowate, gylceryl oleate/cocoate and inorganic salt in particular sodium chloride when especially composition comprise alkyl ether sulphate type of surfactants.

Cleansing compositions of the present invention preferably has a viscosity in the range of 500 to 20,000 mPa.s, more preferably 1,000 to 15,000 mPa.s and most preferably 1,500 to 10,000 mPa.s measured at 20°C with a Brookfield viscosimeter using fro example Spindle 5 at appropriate rotation speed.

The following examples are to illustrate the invention, but not to limit. The compositions according to the invention are prepared by mixing the individual components in water, whereby it is also possible to use pre-mixtures of various ingredients.

### Example 1

| | % by weight |
|---|---|
| Sodium lauryl ether sulphate | 10.6 |
| Cocamidopropyl betaine | 3.0 |
| Sodium lauroyl glutamate | 2.5 |
| Decyl glucoside | 2.0 |
| Palmitamidopropyltrimonium chloride | 0.7 |
| Polyqauternium-10 | 0.5 |
| PEG-90 glyceryl isostearate | 3.0 |
| Citric acid/sodium hydroxide | q.s. to pH 5.5 |
| Preservative, fragrance | q.s |
| Water | to 100 |

The above shampoo was judged to have rich and creamy foam in a monadic test by the volunteers. It was furthermore mentioned that it foams very quickly. Additionally, the hair washed was excellently combable, had good shine, volume and body.

The above cleansing composition was tested against a composition not comprising sodium lauroyl glutamate and palmitamidopropyltrimonium chloride for its colour wash out effect. It was observed that the composition of Example 1 washed out much less colour that the composition not comprising the amino acid and cationic surfactant. It should be noted that the amino acid and cationic surfactants were replaced by anionic and non-ionic surfactants, respectively.

In another test, a composition of Example 1 was tested against a composition not comprising palmitamidopropyltrimonium chloride. The cationic surfactant was replaced with water. It was again observed that the composition of example 1 was eluted much less colour than the comparative composition lacking palmitamidopropyltrimonium chloride.

The colour was out test was carried out at 40°C using coloured hair tresses dipped into 5% solution of cleansing composition which were shaken at a speed of 50 pm. At predetermined times the tresses were taken out and after drying the colour was measured suing laboratory equipment and colour differences were calculated with well known method in the art.

Similar results were observed with the compositions below.

### Example 2

| | % by weight |
|---|---|
| Sodium lauryl ether sulphate | 10.6 |
| Cocamidopropyl hydroxysultaine | 3.0 |
| Sodium lauroyl sacosinate | 2.0 |
| Sodium lauroyl glutamate | 0.8 |
| Decyl glucoside | 2.0 |
| Silicone quaternium-22 | 0.2 |
| Stearamidopropyltrimonium chloride | 0.4 |
| Panthenol | 0.3 |
| PEG-90 glyceryl isostearate | 3.0 |
| Citric acid/sodium hydroxide | q.s. to pH 5.5 |
| Preservative, fragrance | q.s |
| Water | to 100 |

### Example 3

| | % by weight |
|---|---|
| Sodium lauryl ether sulphate | 10.6 |
| Cocamidopropyl betaine | 3.0 |
| Sodium lauroyl sarcosinate | 2.5 |
| Decyl glucoside | 2.0 |
| Polyquaternium-67 | 0.5 |
| Stearamidopropyldimethylamine | 0.5 |
| PEG-90 glyceryl isostearate | 3.0 |
| Lactic acid/sodium hydroxide | q.s. to pH 5.0 |
| Preservative, fragrance | q.s |
| Water | to 100 |

### Example 4

| | % by weight |
|---|---|
| Sodium lauryl ether sulphate | 10.6 |
| Cocamidopropyl betaine | 3.0 |
| Sodium lauroyl glutamate | 2.5 |
| Decyl glucoside | 2.0 |
| Palmitamidopropyltrimonium chloride | 0.3 |
| Silicone quaternium-22 | 0.5 |
| PEG-90 glyceryl isostearate | 3.0 |
| Citric acid/sodium hydroxide | q.s. to pH 5.5 |
| Preservative, fragrance | q.s |
| Water | to 100 |

### Example 5

| | % by weight |
|---|---|
| Sodium lauryl ether sulphate | 10.6 |
| Cocamidopropyl hydroxysultaine | 3.0 |
| Sodium cocoyl sarcosinate | 3.0 |
| Sodium cocoyl glutamate | 0.8 |
| Laureth-16 | 2.0 |
| Palmitamidopropyltrimonium chloride | 0.8 |
| Behenyl alcohol | 0.5 |
| Benzophenone-3 | 0.2 |
| PEG-90 glyceryl isostearate | 3.0 |
| Citric acid/sodium hydroxide | q.s. to pH 5.5 |
| Preservative, fragrance | q.s |
| Water | to 100 |

### Example 6

| | % by weight |
|---|---|
| Sodium lauryl ether sulphate | 10.6 |
| Cocamidopropyl hydroxysultaine | 3.0 |
| Sodium cocoyl glutamate | 0.5 |
| Sodium cocoly sarcosinate | 1.5 |
| Decyl glucoside | 2.0 |
| Guar hydroxypropyltrimonium chloride | 0.5 |
| Palmitamidopropyltrimonium chloride | 0.6 |
| Benzophenone-3 | 0.2 |
| PEG-90 glyceryl isostearate | 3.0 |
| Basic red 51 | 0.1 |
| Basic orange 31 | 0.06 |
| Basic yellow 87 | 0.03 |
| Basic red 76 | 0.08 |
| Lactic acid/sodium hydroxide | q.s. to pH 5.5 |
| Preservative, fragrance | q.s |
| Water | to 100 |

The above shampoo gives hair additionally red shine.

## Claims

1. Aqueous cleansing composition for keratin fibres especially for human hair **characterised in that** it comprises at least one amino acid surfactant selected from glutamate surfactants according to the general formula 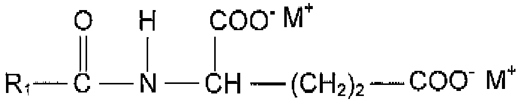 and
sarcosinate surfactants according to the general formula 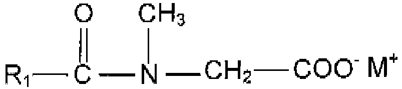 wherein R₁ is saturated or unsaturated, straight or branched alkyl chain with 7 to 17 C atoms, and M is independent from each other H, sodium or potassium at a concentration of 0.25 to 15% by weight, calculated to total composition, and at least one cationic surfactant of the general structure 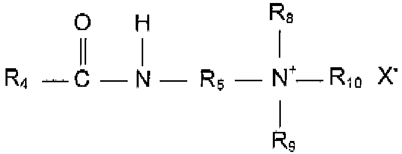 wherein R₄ is a saturated or unsaturated, straight or branched alkyl chain with 8 to 24 C atoms, R₅ is a straight or branched alkyl chain with 1 to 4 C atoms, R₈ and R₉ are the same or different, an alkyl chain with 1 to 4 C atoms, hydroxyl alkyl chain with 1 to 4 C atoms and di hydroxyl alkyl chain with 2 to 4 C atoms, R₁₀ is an alkyl chain with 1 to 4 C atoms, hydroxyl alkyl chain with 1 to 4 C atoms or di hydroxyl alkyl chain with 2 to 4 C atoms and
-R₅-A-R₄
wherein R₄ and R₅ have the above meaning, A is 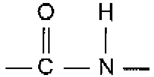 and X is an anion selected from chloride, bromide and methosulfate at a concentration of 0.01 to 5% by weight, calculated to total composition.

2. Process for treating hair wherein hair is treated with a composition comprising at least one hair dyestuff and subsequently optionally washed with a cleansing composition and afterwards treated with an aqueous cleansing composition comprising at least one amino acid surfactant selected from glutamate surfactants according to the general formula 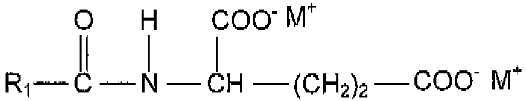 and
sarcosinate surfactants according to the general formula 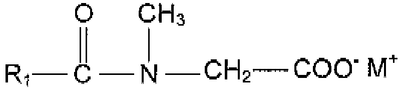 wherein R₁ is saturated or unsaturated, straight or branched alkyl chain with 7 to 17 C atoms, and M is independent from each other H, sodium or potassium at a concentration of 0.25 to 15% by weight, calculated to total of aqueous cleansing composition,
and at least one cationic surfactant of the general structure 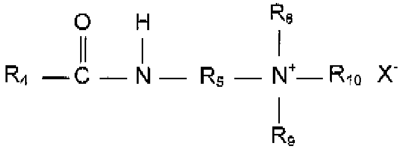 wherein R₄ is a saturated or unsaturated, straight or branched alkyl chain with 8 to 24 C atoms, R₅ is a straight or branched alkyl chain with 1 to 4 C atoms,
R₈ and R₉ are the same or different, an alkyl chain with 1 to 4 C atoms, hydroxyl alkyl chain with 1 to 4 C atoms and di hydroxyl alkyl chain with 2 to 4 C atoms, R₁₀ is an alkyl chain with 1 to 4 C atoms, hydroxyl alkyl chain with 1 to 4 C atoms or di hydroxyl alkyl chain with 2 to 4 C atoms and
-R₅-A-R₄
wherein R₄ and R₅ have the above meaning, A is 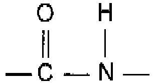 and X is an anion selected from chloride, bromide and methosulfate at a concentration of 0,01 to 5% by weight, calculated to total of aqueous cleansing composition.

3. The process according to claim 2 **characterised in that** the weight ratio of at least one amino acid surfactant according to the general structures to at least one cationic surfactant according to the general structure is at least 1,

4. The process according to claims 2 and 3 **characterised in that** the composition comprises surfactants at a total concentration in the range of 5 to 50% by weight calculated to total composition.

5. The process according to claims 2 to 4 **characterised in that** the composition comprises at least two amino acid surfactants.

6. The process according to claim 5 **characterised in that** the composition comprises mixture of sarcosinate and glutamate surfactants as the amino acid surfactants.

7. The process according to claim 6 **characterised in that** the sarcosinate and glutamate surfactants are comprised in the composition at a weight ratio in the range of 1:1 to 10:1.

8. The process according to claims 2 to 7 **characterised in that** the composition comprises additionally at least one anionic surfactant, preferably selected from the surfactants of the general structure
R₁₁(OCH₂CH₂)ₙOSO₃⁻M⁺
wherein R₁₁ is a straight or branched, saturated or unsaturated alkyl chain with 10 to 24 C atoms, preferably 10 to 18 C atoms, more preferably 10 to 16 C atoms and most preferably 12 to 14 C atoms, n is a value between 1 and 5 and M is a cation such as ammonium, sodium, potassium, magnesium and/or at least one non-ionic surfactant, preferably selected from the non-ionic surfactants according to the general formula
R₁₃-O-(R₁₄O)ₙ-Zₓ,
wherein R₁₃ is an alkyl group with 8 to 18 carbon atoms, R₁₄ is an ethylene or propylene group, Z is a saccharide group with 5 to 6 carbon atoms, n is a number from 0 to 10 and x is a number between 1 and 5 and/or at least one amphoteric surfactant, preferably selected from betaines, amidoalkyl betaines, hydroxysulfobetaines and sulfobetaines, and their mixtures.

9. The process according to claims 2 to 8 **characterised in that** the composition comprises an additional conditioning agent.

10. The process according to claim 9 **characterised in that** the additional conditioning agent is selected form cationic polymers.

11. The process according to claims 2 to 10 **characterised in that** the composition comprises at least one UV filter and/or at least one direct dye.

## Patentansprüche

1. Wässrige Reinigungszusammensetzung für Keratinfasern, insbesondere für menschliche Haare, **dadurch gekennzeichnet, dass** sie mindestens ein Aminosäure-Tensid, ausgewählt aus Glutamat-Tensiden gemäß der allgemeinen Formel 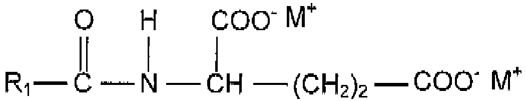 und
Sarcosinat-Tensiden gemäß der allgemeinen Formel 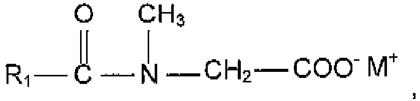 wobei R₁ für eine gesättigte oder ungesättigte, lineare oder verzweigte Alkylkette mit 7 bis 17 C-Atomen steht und M unabhängig voneinander für H, Natrium oder Kalium steht, in einer Konzentration von 0,25 Gewichts-% bis 15 Gewichts-%, bezogen auf die Gesamtzusammensetzung, und mindestens ein kationisches Tensid der allgemeinen Struktur 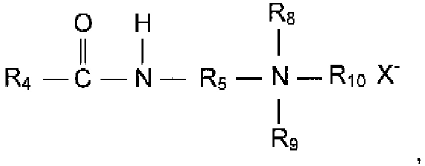 wobei R₄ für eine gesättigte oder ungesättigte, lineare oder verzweigte Alkylkette mit 8 bis 24 C-Atomen steht, R₅ für eine lineare oder verzweigte Alkylkette mit 1 bis 4 C-Atomen steht, R₈ und R₉ gleich oder verschieden sind und für eine Alkylkette mit 1 bis 4 C-Atomen, eine Hydroxyalkylkette mit 1 bis 4 C-Atomen und eine Dihydroxyalkylkette mit 2 bis 4 C-Atomen stehen, R₁₀ für eine Alkylkette mit 1 bis 4 C-Atomen, eine Hydroxyalkylkette mit 1 bis 4 C-Atomen oder eine Dihydroxyalkylkette mit 2 bis 4 C-Atomen und
-R₅-A-R₄
steht, wobei R₄ und R₅ die obige Bedeutung aufweisen, A ist 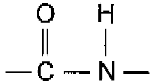 und X für ein Anion steht, ausgewählt aus Chlorid, Bromid und Methosulfat, in einer Konzentration von 0,01 Gewichts-% bis 5 Gewichts-% aufweist, bezogen auf die Gesamtzusammensetzung.

2. Verfahren zum Behandeln von Haaren, wobei die Haare mit einer Zusammensetzung behandelt werden, welche mindestens ein Haarfärbemittel aufweist, und anschließend gegebenenfalls mit einer Reinigungszusammensetzung gewaschen werden und hinterher mit einer wässrigen Reinigungszusammensetzung behandelt werden, welche mindestens ein Aminosäure-Tensid, ausgewählt aus Glutamat-Tensiden gemäß der allgemeinen Formel 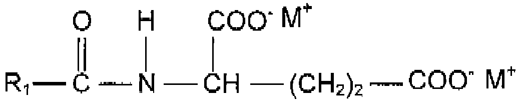 und
Sarcosinat-Tensiden gemäß der allgemeinen Formel 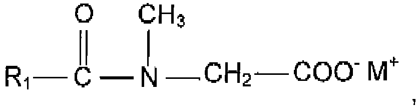 wobei R₁ für eine gesättigte oder ungesättigte, lineare oder verzweigte Alkylkette mit 7 bis 17 C-Atomen steht und M unabhängig voneinander für H, Natrium oder Kalium steht, in einer Konzentration von 0,25 Gewichts-% bis 15 Gewichts-%, bezogen auf die Gesamtzusammensetzung,
und mindestens ein kationisches Tensid der allgemeinen Struktur 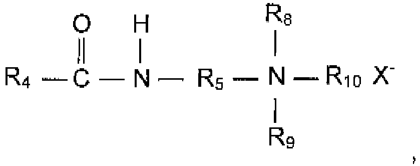 wobei R₄ für eine gesättigte oder ungesättigte, lineare oder verzweigte Alkylkette mit 8 bis 24 C-Atomen steht, R₅ für eine lineare oder verzweigte Alkylkette mit 1 bis 4 C-Atomen steht, R₈ und R₉ gleich oder verschieden sind und für eine Alkylkette mit 1 bis 4 C-Atomen, eine Hydroxyalkylkette mit 1 bis 4 C-Atomen und eine Dihydroxyalkylkette mit 2 bis 4 C-Atomen stehen, R₁₀ für eine Alkylkette mit 1 bis 4 C-Atomen, eine Hydroxyalkylkette mit 1 bis 4 C-Atomen oder eine Dihydroxyalkylkette mit 2 bis 4 C-Atomen und
-R₅-A-R₄
steht, wobei R₄ und R₅ die obige Bedeutung aufweisen, A ist 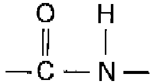 und X für ein Anion steht, ausgewählt aus Chlorid, Bromid und Methosulfat, in einer Konzentration von 0,01 Gewichts-% bis 5 Gewichts-% aufweist, bezogen auf die Gesamtzusammensetzung.

3. Verfahren nach Anspruch 2, **dadurch gekennzeichnet, dass** das Gewichtsverhältnis mindestens eines Aminosäure-Tensids gemäß den allgemeinen Strukturen zu mindestens einem kationischen Tensid gemäß der allgemeinen Struktur mindestens 1 beträgt.

4. Verfahren nach Anspruch 2 und 3, **dadurch gekennzeichnet, dass** die Zusammensetzung Tenside in einer Gesamtkonzentration im Bereich von 5 Gewichts-% bis 50 Gewichts-% aufweist, bezogen auf die Gesamtzusammensetzung.

5. Verfahren nach Anspruch 2 bis 4, **dadurch gekennzeichnet, dass** die Zusammensetzung mindestens zwei Aminosäure-Tenside aufweist.

6. Verfahren nach Anspruch 5, **dadurch gekennzeichnet, dass** die Zusammensetzung ein Gemisch von Sarcosinat- und Glutamat-Tensiden als die Aminosäure-Tenside aufweist.

7. Verfahren nach Anspruch 6, **dadurch gekennzeichnet, dass** die Sarcosinat- und Glutamat-Tenside in einem Gewichtsverhältnis im Bereich von 1:1 bis 10:1 in der Zusammensetzung enthalten sind.

8. Verfahren nach Anspruch 2 bis 7, **dadurch gekennzeichnet, dass** die Zusammensetzung außerdem mindestens ein anionisches Tensid, vorzugsweise ausgewählt aus den Tensiden der allgemeinen Struktur
R₁₁(OCH₂CH₂)ₙOSO₃⁻M⁺,
wobei R₁₁ für eine lineare oder verzweigte, gesättigte oder ungesättigte Alkylkette mit 10 bis 24 C-Atomen, vorzugsweise 10 bis 18 C-Atomen, insbesondere 10 bis 16 C-Atomen und am besten 12 bis 14 C-Atomen steht, n ein Wert von 1 bis 5 ist und M für ein Kation wie Ammonium, Natrium, Kalium, Magnesium steht, und/oder mindestens ein nichtionisches Tensid, vorzugsweise ausgewählt aus den nichtionischen Tensiden gemäß der allgemeinen Formel
R₁₃-O-(R₁₄O)ₙ-Zₓ,
wobei R₁₃ für eine Alkylgruppe mit 8 bis 18 Kohlenstoffatomen steht, R₁₄ für eine Ethylen- oder Propylengruppe steht, Z für eine Saccharidgruppe mit 5 bis 6 Kohlenstoffatomen steht, n eine Zahl von 0 bis 10 ist und x eine Zahl von 1 bis 5 ist, und/oder mindestens ein amphoteres Tensid aufweist, vorzugsweise ausgewählt aus Betainen, Amidoalkylbetainen, Hydroxysulfobetainen und Sulfobetainen und deren Gemischen.

9. Verfahren nach Anspruch 2 bis 8, **dadurch gekennzeichnet, dass** die Zusammensetzung zusätzlich ein Konditionierungsmittel aufweist.

10. Verfahren nach Anspruch 9, **dadurch gekennzeichnet, dass** das zusätzliche Konditionierungsmittel aus kationischen Polymeren ausgewählt ist.

11. Verfahren nach Anspruch 2 bis 10, **dadurch gekennzeichnet, dass** die Zusammensetzung mindestens einen UV-Filter und/oder mindestens einen Direktfarbstoff aufweist.

## Revendications

1. Composition aqueuse de nettoyage pour fibres de kératine, en particulier pour les cheveux humains, **caractérisée en ce qu'**elle comprend au moins un tensioactif de type acide aminé choisi parmi des tensioactifs de type glutamate selon la formule générale 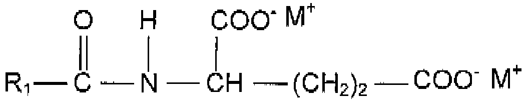 et
des tensioactifs de type sarcosinate selon la formule générale 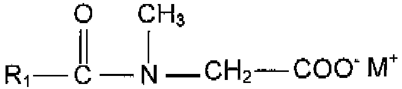 où R₁ est une chaîne alkyle saturée ou non saturée, linéaire ou ramifiée, avec de 7 à 17 atomes C, et M est indépendamment l'un de l'autre un ion d'H, un ion sodium ou un ion potassium à une concentration en poids entre 0,25 et 15 %, calculée par rapport à la composition totale, et au moins un tensioactif cationique selon la structure générale 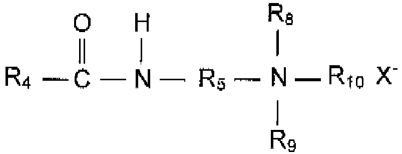 où R₄ est une chaîne alkyle saturée ou non saturée, linéaire ou ramifiée, avec de 8 à 24 atomes C, R₅ est une chaîne alkyle linéaire ou ramifiée avec de 1 à 4 atomes C,
R₈ et R₉ sont, identiques ou différents, une chaîne alkyle avec de 1 à 4 atomes C, une chaîne hydroxyalkyle avec de 1 à 4 atomes C et une chaîne di-hydroxyalkyle avec de 2 à 4 atomes C, R₁₀ est une chaîne alkyle avec de 1 à 4 atomes C, une chaîne hydroxyalkyle avec de 1 à 4 atomes C ou une chaîne di-hydroxyalkyle avec de 2 à 4 atomes C, et un groupe
-R₅-A-R₄
où R₄ et R₅ ont la signification qui a été donnée ci-dessus, A est 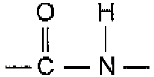 et X est un anion choisi parmi le chlorure, le bromure et le méthosulfate, à une concentration en poids entre 0,01 et 5%, calculée par rapport à la composition totale.

2. Procédé de traitement des cheveux dans lequel les cheveux sont traités avec une composition comprenant au moins une teinture pour cheveux et sont ensuite éventuellement lavés avec une composition de nettoyage et a postériori traités avec une composition de nettoyage aqueuse comprenant au moins un tensioactif de type acide aminé choisi parmi les tensioactifs de type glutamate selon la formule générale 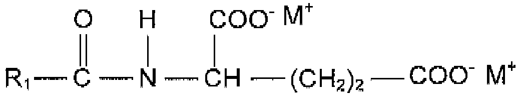 et
des tensioactifs de type sarcosinate selon la formule générale 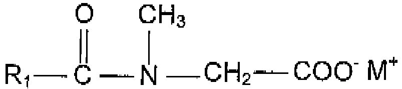 où R₁ est une chaîne alkyle saturée ou non saturée, linéaire ou ramifiée, avec de 7 à 17 atomes C, et M est indépendamment l'un de l'autre un ion d'H, un ion sodium ou potassium à une concentration en poids entre 0,25 et 15 %, calculée par rapport à la totalité de la composition aqueuse de nettoyage,
et au moins un tensioactif cationique selon la structure générale 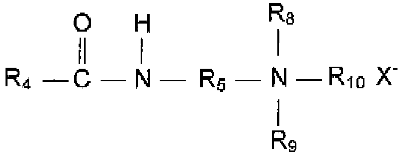 où R₄ est une chaîne alkyle saturée ou non saturée, linéaire ou ramifiée, avec de 8 à 24 atomes C, R₅ est une chaîne alkyle linéaire ou ramifiée avec de 1 à 4 atomes C,
R₈ et R₉ sont, identiques ou différents, une chaîne alkyle avec de 1 à 4 atomes C, une chaîne hydroxyalkyle avec de 1 à 4 atomes C et une chaîne di-hydroxyalkyle avec de 2 à 4 atomes C, R₁₀ est une chaîne alkyle avec de 1 à 4 atomes C, une chaîne hydroxyalkyle avec de 1 à 4 atomes C ou une chaîne di-hydroxyalkyle avec de 2 à 4 atomes C et un groupe
-R₅-A-R₄
où R₄ et R₅ ont la signification qui a été donnée ci-dessus, A est 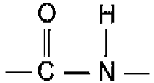 et X est un anion choisi parmi le chlorure, le bromure et le méthosulfate à une concentration en poids entre 0,01 et 5%, calculée par rapport à la totalité de la composition aqueuse de nettoyage.

3. Procédé selon la revendication 2, **caractérisé en ce que** le ratio pondéral entre au moins un tensioactif de type acide aminé selon les structures générales et au moins un tensioactif cationique selon la structure générale est au moins de 1.

4. Procédé selon les revendications 2 et 3, **caractérisé en ce que** la composition comprend des tensioactifs à une concentration totale en poids située dans une plage entre 5 et 50 %, calculée par rapport à la composition totale.

5. Procédé selon les revendications 2 à 4, **caractérisé en ce que** la composition comprend au moins deux tensioactifs de type acide aminé.

6. Procédé selon la revendication 5, **caractérisé en ce que** la composition comprend un mélange de tensioactifs de type sarcosinate et de type glutamate en tant que tensioactifs de type acide aminé.

7. Procédé selon la revendication 6, **caractérisé en ce que** les tensioactifs de type sarcosinate et de type glutamate sont compris dans la composition dans un ratio pondéral situé dans une plage entre 1 : 1 et 10 : 1.

8. Procédé selon les revendications 2 à 7, **caractérisé en ce que** la composition comprend par ailleurs au moins un tensioactif anionique, de préférence choisi parmi des tensioactifs selon la structure générale
R₁₁(OCH₂CH₂)ₙOSO₃⁻M⁺
où R₁₁ est une chaîne alkyle linéaire ou ramifiée, saturée ou non saturée avec de 10 à 24 atomes C, de préférence de 10 à 18 atomes C, plus préférentiellement de 10 à 16 atomes C et idéalement de 12 à 14 atomes C, n est une valeur entre 1 et 5 et M est un cation, comme, par exemple, un cation ammonium, un cation sodium, un cation potassium, un cation magnésium, et/ou au moins un tensioactif non ionique, de préférence choisi parmi des tensioactifs non ioniques selon la formule générale
R₁₃-O-(R₁₄O)ₙ-Zₓ,
où R₁₃ est un groupement alkyle avec de 8 à 18 atomes de carbone, R₁₄ est un groupement éthylène ou propylène, Z est un groupement saccharide avec de 5 à 6 atomes de carbone, n est un nombre entre 0 et 10 et x est un nombre entre 1 et 5, et/ou au moins un tensioactif amphotère, de préférence choisi parmi des bétaïnes, des amidoalkyl bétaïnes, des hydroxysulfobétaïnes et des sulfobétaïnes, et leurs mélanges.

9. Procédé selon les revendications 2 à 8, **caractérisé en ce que** la composition comprend un agent de conditionnement supplémentaire.

10. Procédé selon la revendication 9, **caractérisé en ce que** l'agent de conditionnement supplémentaire est choisi parmi des polymères cationiques.

11. Procédé selon les revendications 2 à 10, **caractérisé en ce que** la composition comprend au moins un filtre UV et/ou au moins un colorant direct.
